# EUROPEAN PATENT APPLICATION

(11) **EP 1 532 971 A1**
(43) Date of publication of application: **25.05.2005**
(21) Application number: 02733375.6
(22) Date of filing: 07.06.2002
(51) Int. Cl.: A61K 7/16

(54) **COMPOSITIONS FOR ORAL CAVITY**

(71) Applicant: SUNSTAR KABUSHIKI KAISHA, Takatsuki-shi, Osaka 569-1195 (JP)
(72) Inventor: NAKAO, Akira, c/o Hamuro R & D Center SUNSTAR K.K., Takatsuki-shi, Osaka 569-1044 (JP); SAITO, Toru, c/o Hamuro R & D Center SUNSTAR K.K., Takatsuki-shi, Osaka 569-1044 (JP)
(74) Representative: Stoner, Gerard Patrick
(86) International application number: PCT/JP2002/005639
(87) International publication number: WO 2003/103618

(57) **Abstract**

The present invention provides an oral composition comprising microcrystalline cellulose, and one or more surface active agents selected from the group consisting of alkyl glucoside, polyglycerin fatty acid ester, sucrose fatty acid ester and betaine, which has an excellent shape-holding ability and dispersibility in an oral cavity, does not change a taste of juice after teeth brushing, in addition, has excellent stability with time not causing solid-liquid separation. Moreover, the present invention provides an oral composition comprising a cationic antimicrobial agent and microcrystalline cellulose, which can effectively prevent an oral cavity disease such as a periodontal disease, dental caries and the like by enhancing an ability of the cationic antimicrobial agent to reside on a tooth surface.

## Description

### Background of the Invention

### Field of the Invention

The present invention relates to an oral composition which has an excellent shape-holding ability and dispersibility, and does not change a taste of juice after teeth brushing and, particularly, has excellent stability with time. Moreover, the present invention relates to an oral composition having an excellent ability of a cationic antimicrobial agent to reside on a tooth surface.

### Description of the Related Art

Hitherto, a shape-holding ability and dispersibility in an oral cavity of an oral composition have been obtained by containing therein a thickening agent, which is generally and frequently used, such as carboxymethyl cellulose, methyl cellulose, hydroxyethyl cellulose, gum arabic, xanthan gum, carrageenan, sodium alginate, sodium polyacrylate and the like. In addition, an oral composition having better shape-holding ability and dispersibility in an oral cavity than those of prior art, which is prepared by containing therein finely-divided cellulose, has been proposed in JPA 58861/1993. However, such the oral composition has practical problems such as occurrence of solid-liquid separation during long term storage. In addition, in such the oral composition, sodium alkylsulfate is used as a surface active agent, which is known to change a taste of juice after teeth brushing.

On the other hand, a cationic antimicrobial agent is contained in various oral compositions in order to prevent an oral cavity disease such as a periodontal disease, dental caries and the like, because it has an excellent ability to be adsorbed to an oral tissue, an enhanced antimicrobial activity and an enhanced plaque formation-suppressing effect.

However, there was a problem that, since the cationic antimicrobial agent has an electric charge, it forms an electrostatic complex with other anionic ingredients contained in the oral composition, and an antimicrobial activity per unit of the cationic antimicrobial agent is reduced. In response thereto, attempts have been conducted to prevent reduction of the activity per unit of the cationic antimicrobial agent, by containing a nonionic or amphoteric surface active agent or a nonionic thickening agent in the oral compositions, but sufficient effects have not been obtained yet.

On the other hand, even the cationic antimicrobial agent exhibits a transient antimicrobial effect in many cases, and it is contemplated that an activity of the cationic antimicrobial agent can be totally enhanced by improving an ability of the cationic antimicrobial agent to reside on a tooth surface.

### Brief Summary of the Invention

A first object of the present invention is to provide an oral composition which retains better shape-holding ability, is excellent in dispersibility in an oral cavity, does not change a taste of juice after teeth brushing, and does not cause solid-liquid separation during long term storage and, additionally, which has an improved ability of the cationic antimicrobial agent to reside on a tooth surface.

Moreover, a second object of the present invention is to provide an oral composition which can effectively prevent a periodontal disease and dental caries by enhancing the ability of a cationic antimicrobial agent to reside on a tooth surface to enhance a residence antimicrobial activity of the cationic antimicrobial agent.

In view of above former situations, the present inventors studied intensively, and found that an oral composition which has an excellent shape-holding ability and dispersibility in an oral cavity, does not change a taste of juice after teeth brushing, and does not cause solid-liquid separation during long term storage, can be obtained by containing a combination of microcrystalline cellulose and a particular surface active agent, which resulted in completion of a first aspect of the present invention.

Moreover, in view of above latter situations, the present inventors studied intensively, and found that the ability of the cationic antimicrobial agent to reside on a tooth surface is significantly enhanced by containing a specific combination of the cationic antimicrobial agent and microcrystalline cellulose, which resulted in completion of a second aspect of the present invention.

That is, in accordance with the first aspect, the present invention provides:
1. An oral composition comprising microcrystalline cellulose, and one or more surface active agents selected from the group consisting of alkyl glucoside, polyglycerin fatty acid ester, sucrose fatty acid ester and betaine;
2. The oral composition of according to (1), wherein the microcrystalline cellulose is contained at 0.2-10 % by weight;
3. The oral composition according to (1) or (2), wherein the surface active agent is alkyl glucoside;
4. The oral composition according to (3), wherein an alkyl chain of the alkyl glucoside is C8-C16 in length;
5. The oral composition according to (1) or (2), wherein the surface active agent is polyglycerin fatty acid ester or sucrose fatty acid ester;
6. The oral composition according to (5), wherein an alkyl chain of a fatty acid portion of the polyglycerin fatty acid ester or the sucrose fatty acid ester is C8-C16 in length;
7. The oral composition according to (1) or (2), wherein the surface active agent is betaine;
8. The oral composition according to (7), wherein the betaine is fatty acid amide propyl betaine;
9. The oral composition according to (8), wherein an alkyl chain of a fatty acid portion of the fatty acid amide propyl betaine is C8-C16 in length; and
10. The oral composition according to any one of (1)-(9), further comprising a cationic antimicrobial agent.

According to the first aspect of the present invention, an oral composition can be provided, which has an excellent shape-holding ability and dispersibility in an oral cavity, does not change a taste of juice after teeth brushing and, particularly, has excellent stability with time, or additionally has an enhanced ability of the cationic antimicrobial agent to reside on a tooth surface in addition to above characteristics.

Moreover, in accordance with the second aspect, the present invention provides:
11. An oral composition comprising a cationic antimicrobial agent and microcrystalline cellulose;
12. The oral composition according to (11), wherein the cationic antimicrobial agent is a quaternary ammonium salt;
13. The oral composition according to (11), wherein the cationic antimicrobial agent is a biguanide antimicrobial agent;
14. The oral composition according to (11), wherein the cationic antimicrobial agent is one or more selected from the group consisting of cetylpyridinium chloride, benzalkonium chloride, benzethonium chloride, chlorhexidine hydrochloride and chlorhexidine gluconate;
15. The oral composition according to any one of (11)-(14), wherein the cationic antimicrobial agent is contained at 0.001-10 % by weight;
16. The oral composition according to any one of (11)-(15), wherein the microcrystalline cellulose is contained at 0.2-10 % by weight;
17. The oral composition according to any one of (11)-(16), further comprising one or more surface active agents selected from nonionic and amphoteric surface active agents;
18. The oral composition according to (17), wherein the surface active agent is alkyl glucoside having an alkyl chain of C8-C16 in length; and
19. The oral composition according to (17), wherein the surface active agent is fatty acid amide propyl betaine having an alkyl chain of a fatty acid portion of C8-C16 in length.

According to the second aspect of the present invention, an oral composition can be provided, which can significantly enhance the effect of the cationic antimicrobial agent to reside on the tooth surface and effectively prevent the oral cavity disease such as the periodontal disease, dental caries and the like.

### Detailed Description of the Invention

The first and second aspects of the present invention are sequentially illustrated below.

Microcrystalline cellulose used in the first aspect of the present invention is not particularly limited as far as it is commercially available, but microcrystalline cellulose having an average particle diameter of 10 micrometer or smaller is more preferable and microcrystalline cellulose having an average particle diameter of 2-6 micrometer is most preferable. When the average particle diameter of microcrystalline cellulose is larger than 10 micrometer, dispersibility of the oral composition in the oral cavity is deteriorated. In addition, an amount of microcrystalline cellulose to be contained is preferably 0.2-10 % by weight based on a total weight of the oral composition. When the amount of microcrystalline cellulose is smaller than 0.2 % by weight, an adequate shape-holding ability of the oral composition can not be achieved, being is not preferable. On the other hand, when the amount of microcrystalline cellulose is larger than 10 % by weight, a viscosity of the oral composition becomes too high, being not preferable.

The surface active agent used in the first aspect of the present invention includes alkyl glucoside, polyglycerin fatty acid ester, sucrose fatty acid ester and betaine, and they may be used alone or in a combination of two or more. An amount of the surface active agent to be contained is preferably 0.5-5 % by weight based on a total weight of the oral composition. When the amount of the surface active agent to be contained is smaller than 0.5 % by weight, a foaming ability of the oral composition is reduced, and a use feeling is deteriorated, being not preferable. On the other hand, when the amount of the surface active agent to be contained is larger than 5 % by weight, a taste or a smell derived from the surface active agent becomes unnegligible, being not preferable.

Among above surface active agents, alkyl glucoside used in the present invention is not particularly limited, but an alkyl chain thereof is preferably C8-C16 in length. When the alkyl chain is shorter than C8, a bitter taste is produced in the oral composition, being not preferable. On the other hand, when the alkyl chain is longer than C16, the foaming ability of the oral composition is lowered and it becomes uncomfortable to use in some cases, being not preferable. Examples within such the chain length range include decyl glucoside, lauryl glucoside, myristyl glucoside and the like, and PLANTACARE 1200, PLANTACARE 2000 (Cognis), Oramix NS10, Oramix NS26 (SEPPIC) and the like are commercially available.

In addition, polyglycerin fatty acid ester used in the first aspect of the present invention is not particularly limited, but an alkyl chain of a fatty acid portion thereof is preferably C8-C16 in length. When the alkyl chain is shorter than C8, a bitter taste is produced in the oral composition. On the other hand, when the alkyl chain is longer than C16, there is a tendency that the foaming ability of the oral composition is lowered. In addition, a polymerization degree of a polyglycerin portion is preferably equal to or greater than 4. When the polymerization degree is equal to or smaller than 3, there is a tendency that the foaming ability of the oral composition is lowered. Examples of such polyglycerin fatty acid ester include decaglycerin monolauric acid ester, tetraglycerin monolauric acid ester, decaglycerin monomyristic acid ester, tetraglycerin monomyristic acid ester and the like, and NIKKOL Decaglyn 1-L, NIKKOL Tetraglyn 1-L, NIKKOL Decaglyn 1-M (Nikko Chemicals, Co., Ltd.), Sunsoft Q-12W, Sunsoft Q-12T, Sunsoft Q-14W (Taiyo Kagaku Co., Ltd.) and the like are commercially available.

In addition, sucrose fatty acid ester used in the first aspect of the present invention is not particularly limited, but an alkyl chain of a fatty acid portion thereof is preferably C8-C16 in length. When the alkyl chain is shorter than C8, a bitter taste is produced in the oral composition. On the other hand, when the alkyl chain is longer than C16, the foaming ability of the oral composition is lowered and an oily taste is produced in some cases in the oral composition. Examples of such sucrose fatty acid ester include sucrose lauric acid ester, sucrose palmitic acid ester and the like, and DK ester S series (Daiichi Kogyo Seiyaku Co., Ltd.), Ryoto sugar ester (Mitsubishi Kagaku Foods Co.) and the like are commercially available.

In addition, the betaine surface active agent used in the first aspect of the present invention is not particularly limited, but examples thereof include alkyl betaine, fatty acid amide propyl betaine, alkyl sulfobetaine, imidazolinium betaine and the like. Among them, fatty acid amide propyl betaine is preferable in view of its weak bitter taste. In addition, an alkyl chain of a fatty acid portion of fatty acid amide propyl betaine is preferably C8-C16 in length. When the alkyl chain is shorter than C8, a bitter taste is produced in the oral composition. On the other hand, when the alkyl chain is longer than C16, the foaming ability of the oral composition is lowered and an oily taste is produced in some cases in the oral composition. Examples of fatty acid amide propyl betaine having such the chain length range include cocamidopropyl betaine, lauric acid amide propyl betaine, myristic acid amide propyl betaine and the like, and there are commercially available products such as SWANOL (Nikko Chemicals, Co., Ltd.), Obazolin (Toho Chemical Industry Co., Ltd.), RIKABION (New Japan Chemical Co., Ltd.), Tego-Betaine (Goldschmidt AG), Empigen (Albright & Wilson) and the like.

In addition, a cationic antimicrobial agent used in the first aspect of the present invention is not particularly limited, but a quaternary ammonium salt and a biguanide antimicrobial agent are preferable, and examples thereof include, for example, the quaternary ammonium salt such as cetylpyridinium chloride, benzalkonium chloride, benzethonium chloride, distearyldimethyl ammonium chloride, stearyldimethylbenzyl ammonium chloride, stearyltrimethyl ammonium chloride, cetyltrimethyl ammonium chloride, lauryltrimethyl ammonium chloride, laurylpyridinium chloride and the like, and the biguanide antimicrobial agent such as chlorhexidine hydrochloride, chlorhexidine acetate, chlorhexidine gluconate, alexidin hydrochloride, alexidin acetate, alexidin gluconate and the like, and the like. Among them, cetylpyridinium chloride and benzalkonium chloride are more preferable, and cetylpyridinium chloride is particularly preferable. These cationic bacrtericides may be contained alone or in a combination of two or more. In addition, an mount of the cationic antimicrobial agent to be contained is preferably 0.001-10 % by weight, and more preferably 0.01-1 % by weight based on a total weight of the oral composition. When the amount of the cationic antimicrobial agent is smaller than 0.001 % by weight, an antimicrobial effect of the oral composition can not be expected. On the other hand, when the amount of the cationic antimicrobial agent is larger than 10 % by weight, an irritation to an oral mucous membrane becomes strong, being not preferable in view of safety.

The oral composition of the first aspect of the present invention can be prepared in a form of toothpaste, wet dentifrices, liquid dentifrices, oral paste, gels, sprays, foams and the like. Ingredients, for example, active ingredients, foaming agents or detergents, polishing agents, thickening agents, humectants, preservatives, flavors, sweeteners, pH adjusting agents or the like may be properly contained in the oral composition of the first aspect of the present invention as far as they do not deteriorate the effects of the present invention, depending on a difference in the form of the oral composition.

Among them, examples of the active ingredient include a nonionic antimicrobial agent such as triclosan, isopropyl methylphenol and the like, a fluoride such as sodium fluoride, potassium fluoride, ammonium fluoride, stannous fluoride, sodium monofluorophosphate and the like, an enzyme such as amylase, protease, lysozyme, dextranase and the like, a vitamin such as vitamins B, C and E and the like, a potassium salt and the like.

Examples of the foaming agent or detergent include an anionic surface active agent such as sodium N-acyl sarcosinate, N-acyl glutamate, sodium N-methyl-N-acyltaurine, sodium N-methyl-N-acylalanine, sodium alpha-olefin sulfonate and the like; a nonionic surface active agent such as polyoxyethylene fatty acid ester such as polyoxyethylene sorbitan fatty acid ester such as polyoxyethylene sorbitan monolaurate, or polyoxyethylene hydrogenated castor oil, lauric acid monoethanol amide, myristic acid monoethanol amide, polyoxyethylene higher alcohol-ether, polyoxyethylene (polyoxypropylene) copolymer, polyoxyethylene (polyoxypropylene) fatty acid ester and the like; an amphoteric surface active agent such as N-alkyldiamino ethyl glycine and the like, in addition to the surface active agents as described above. But, when the oral composition of the first aspect of the present invention contains the cationic antimicrobial agent, it is not preferable that it contains the anionic surface active agent.

Examples of the polishing agent include calcium hydrogenphosphate dihydrate or anhydrate, calcium phosphate, calcium tertiary phosphate, magnesium tertiary phosphate, calcium pyrophosphate, hydroxyapatite, insoluble sodium metaphosphate, silicic acid hydrate, silicic acid anhydrate, silica gel, precipitated silica, aluminum silicate, zirconium silicate, calcium silicate, calcium carbonate, magnesium carbonate, alumina, aluminum hydroxide, calcium sulfate, methyl polymethacrylate and the like.

Examples of the thickening agent include an anionic thickening agent such as sodium carboxymethyl cellulose, sodium carboxymethyl hydroxyethyl cellulose and the like, a cellulose derivative such as hydroxyethyl cellulose, hydroxypropyl cellulose and the like, natural gum such as xanthan gum, tragacanth, gum karaya, gum arabic, carrageenan and the like, a cationic thickening agent such as O-[2-hydroxy-3-(trimethylammonio) propyl]hydroxyethyl cellulose chloride and the like, in addition to microcrystalline cellulose used in the present invention. But, when the oral composition of the first aspect of the present invention contains the cationic antimicrobial agent, it is not preferable that it contains the anionic thickening agent.

Examples of the humectant include glycerin, propylene glycol, 1,3-butylene glycol, sorbitol, polyethylene glycol, xylitol, polypropylene glycol and the like.

Examples of the preservative include paraoxybenzoic acid ester such as methyl paraben, propyl paraben and the like, benzoate, sodium benzoate and the like.

Examples of the flavor include menthol, carvone, eugenol, methyl salicylate, methyl eugenol, thymol, anethole, limonene, ocimene, n-decyl alcohol, citronel, alpha-terpineol, methyl acetate, citronenyl acetate, cinneole, linalool, ethyl linalool, vanillin, thyme, nutmeg, spearmint oil, peppermint oil, lemon oil, orange oil, sage oil, rosemary oil, cinnamon oil, perilla oil, wintergreen oil, cloves oil, eucalyptus oil, piment oil, tea tree oil, Davana oil and the like.

Examples of the sweetener include saccharin sodium, acesulfame potassium, stevioside, neohesperidin dihydrochalcone, glycyrrhizin, perillartine, thaumatin, aspartyl phenylalanine methyl ester, methoxycinnamic aldehyde, xylit and the like.

Examples of the pH-adjusting agent include citric acid, phosphoric acid, malic acid, gluconic acid, maleic acid, aspartic acid, gluconic acid, succinic acid, glucuronic acid, fumaric acid, glutamic acid, adipic acid and salts thereof, hydrochloric acid, sodium hydroxide, potassium hydroxide, sodium silicate and the like.

These ingredients may be contained alone or in a combination of two or more in the oral composition of the first aspect of the present invention.

Next, the cationic antimicrobial agent used in the second aspect of the present invention is not particularly limited, but a quaternary ammonium salt and a biguanide antimicrobial agent are preferable, and examples thereof include, for example, the quaternary ammonium salt such as cetylpyridinium chloride, benzalkonium chloride, benzethonium chloride, distearyldimethyl ammonium chloride, stearyldimethylbenzyl ammonium chloride, stearyltrimethyl ammonium chloride, cetyltrimethyl ammonium chloride, lauryltrimethyl ammonium chloride, laurylpyridinium chloride and the like, and the biguanide antimicrobial agent such as chlorhexidine hydrochloride, chlorhexidine acetate, chlorhexidine gluconate, alexidin hydrochloride, alexidin acetate, alexidin gluconate and the like, and the like. These cationic antimicrobial agents may be contained alone or in a combination of two or more. In addition, an amount of the cationic antimicrobial agent to be contained is preferably 0.001-10 % by weight and more preferably 0.01-1 % by weight based on a total weight of the oral composition. When the amount of the cationic antimicrobial agent is smaller than 0.001 % by weight, an expected antimicrobial effect is not exerted. On the other hand, when the amount of the cationic antimicrobial agent is larger than 10 % by weight, an irritation to an oral mucous membrane becomes strong, being not preferable in view of safety.

In addition, microcrystalline cellulose used in the second aspect of the present invention is not particularly limited as far as it is commercially available. An amount of microcrystalline cellulose to be contained is preferably 0.2-10 % by weight and more preferably 0.5-5 % by weight based on a total weight of the oral composition. When the amount of microcrystalline cellulose is smaller than 0.2 % by weight, an effect for enhancing residence of the antimicrobial agent on a tooth surface is lowered. On the other hand, when the amount of microcrystalline cellulose is larger than 10 % by weight, a viscosity of the oral composition becomes too high, being not preferable. In addition, an average particle diameter of microcrystalline cellulose is preferably equal to or smaller than 10 micrometer and more preferably 2-6 micrometer, in view of homogeneous dispersion in the oral composition. In addition, as a matter of fact, microcrystalline cellulose having an average particle diameter smaller than 0.1 micrometer is hard to obtain.

In addition, the surface active agent used in the second aspect of the present invention is preferably a nonionic surface active agent, an amphoteric surface active agent or a cationic surface active agent. When the anionic surface active agent is used in the oral composition of the present invention, stability of the cationic antimicrobial agent in a formulation may be deteriorated. More preferably, the surface active agent is the nonionic and amphoteric surface active agents. Examples of the nonionic surface active agent include, for example, sugar fatty acid ester such as alkyl glucoside, sucrose fatty acid ester, maltose fatty acid ester, lactose fatty acid ester and the like, polyoxyethylene alkyl ether, fatty acid alkanol amide, polyoxyethylene sorbitan fatty acid ester such as polyoxyethylene sorbitan monolaurate, polyoxyethylene sorbitan monostearate and the like, polyoxyethylene hydrogenated castor oil, sorbitan fatty acid ester, polyglycerin fatty acid ester such as decaglycerin monolauric acid ester, pentaglycerin distearic acid ester and the like, polyoxyethylene (polyoxypropylene) copolymer, and the like. Examples of the amphoteric surface active agent include, for example, N-alkyldiamino ethyl glycine such as N-lauryldiamino ethyl glycine, N-myristyl dimino ethyl glycine and the like, fatty acid amide propyl betaine, N-alkyl-N-carboxymethyl ammonium betaine, sodium 2-alkyl-1-hydroxyethyl imidazoline betaine and the like. Among them, alkyl glucoside, sucrose fatty acid ester, polyoxyethylene hydrogenated caster oil, polyglycerin fatty acid ester, polyoxyethylene (polyoxypropylene) copolymer, N-alkyl diamino ethyl glycine and fatty acid amide propyl betaine are preferable. Among them, alkyl glucoside and fatty acid amide propyl betaine are particularly preferable. In addition, an alkyl chain of alkyl glucoside of C8-C16 in length is preferable, and an alkyl chain of alkyl glucoside of C10-C14 in length is particularly preferable. In addition, an alkyl chain of a fatty acid portion of fatty acid amide propyl betaine is preferably C10-C14 in length, and particularly C12-C14 in length. An amount of the surface active agent to be contained is preferably 0.5-5 % by weight based on a total weight of the oral composition.

The oral composition of the second aspect of the present invention can be prepared in a form of toothpaste, wet dentifrices, liquid dentifrices, oral paste, gels and the like. Ingredients, for example, active ingredients, polishing agents, thickening agents, humectants, preservatives, flavors, sweeteners, pH adjusting agents or the like may be properly contained in the oral composition of the second aspect of the present invention as far as they do not deteriorate the effects of the present invention, depending on a difference in the form of the oral composition.

Among them, examples of the active ingredient include a nonionic antimicrobial agent such as triclosan, isopropyl methylphenol and the like, a fluoride such as sodium fluoride, potassium fluoride, ammonium fluoride, stannous fluoride, sodium monofluorophosphate and the like, an enzyme such as amylase, protease, lysozyme, dextranase and the like, a vitamin such as vitamins B, C and E and the like, an astringent such as potassium nitrate, aluminum lactate and the like, and the like, in addition to the cationic antimicrobial agent such as the quaternary ammonium salt and the biguanide antimicrobial agent as described above.

Examples of the polishing agent include calcium hydrogenphosphate, dihydrate and anhydrate, calcium phosphate, calcium tertiary phosphate, magnesium tertiary phosphate, calcium pyrophosphate, hydroxyapatite, insoluble sodium metaphosphate, silicic acid hydrate, silicic acid anhydrate, silica gel, precipitated silica, aluminum silicate, zirconium silicate, calcium silicate, calcium carbonate, magnesium carbonate, alumina, aluminum hydroxide, calcium sulfate, methyl polymethacrylate and the like. Among them, calcium hydrogenphosphate, dihydrate and anhydrate, calcium phosphate, calcium tertiary phosphate, magnesium tertiary phosphate, calcium pyrophosphate, hydroxyapatite, calcium carbonate and magnesium carbonate are preferable.

Examples of the thickening agent include a cellulose derivative such as hydroxyethyl cellulose, hydroxypropyl cellulose and the like, a natural gum such as carrageenan, xanthan gum, tragacanth, gum karaya, gum arabic, gellan gum and the like, a synthetic thickening agent such as poly (vinylalcohol), sodium polyacrylate and the like, an inorganic thickening agent such as viscosity-increasing silica, veegum and the like, and the like, in addition to microcrystalline cellulose used in the oral composition of the present invention.

Examples of the humectant include glycerin, ethylene glycol, propylene glycol, 1,3-butylene glycol, polyethylene glycol, polypropylene glycol, sorbit, xylit, maltit, lactit, palatinit and the like.

Examples of the preservative include paraoxybenzoic acid ester such as methyl paraben, propyl paraben and the like, benzoate, sodium benzoate and the like.

Examples of the flavor include menthol, carvone, eugenol, methyl salicylate, methyl eugenol, thymol, anethole, limonene, ocimene, n-decyl alcohol, citronel, alpha-terpineol, methyl acetate, citronenyl acetate, cinneole, linalool, ethyl linalool, vanillin, thyme, nutmeg, spearmint oil, peppermint oil, lemon oil, orange oil, sage oil, rosemary oil, cinnamon oil, perilla oil, wintergreen oil, cloves oil, eucalyptus oil, piment oil, tea tree oil, Davana oil and the like.

Examples of the sweetener include saccharin sodium, acesulfame potassium, stevioside, neohesperidin dihydrochalcone, glycyrrhizin, perillartine, thaumatin, aspartyl phenylalanine methyl ester, methoxycinnamic aldehyde, xylit and the like.

Examples of the pH-adjusting agent include citric acid, phosphoric acid, malic acid, gluconic acid, maleic acid, aspartic acid, gluconic acid, succinic acid, glucuronic acid, fumaric acid, glutamic acid, adipic acid and salts thereof, hydrochloric acid, sodium hydroxide, potassium hydroxide, sodium silicate and the like.

These ingredients may be contained alone or in a combination of two or more in the oral composition of the second aspect of the present invention.

### Examples

The first and second aspects of the present invention will be further illustrated in detail by referring to the following Examples, but the present invention is not limited to such the Examples. In the Examples, the term "%" means "% by weight", unless otherwise indicated.

Each oral composition of the present invention was prepared according to the formulation shown in Table 1 by conventional procedures. Each composition obtained was tested for stability with time at room temperature for one month. The results thereof are shown in Table 1.

### Evaluation criteria

Stability with time after one month storage at room temperature:
O: No solid-liquid separation was observed
X: Solid-liquid separation was observed

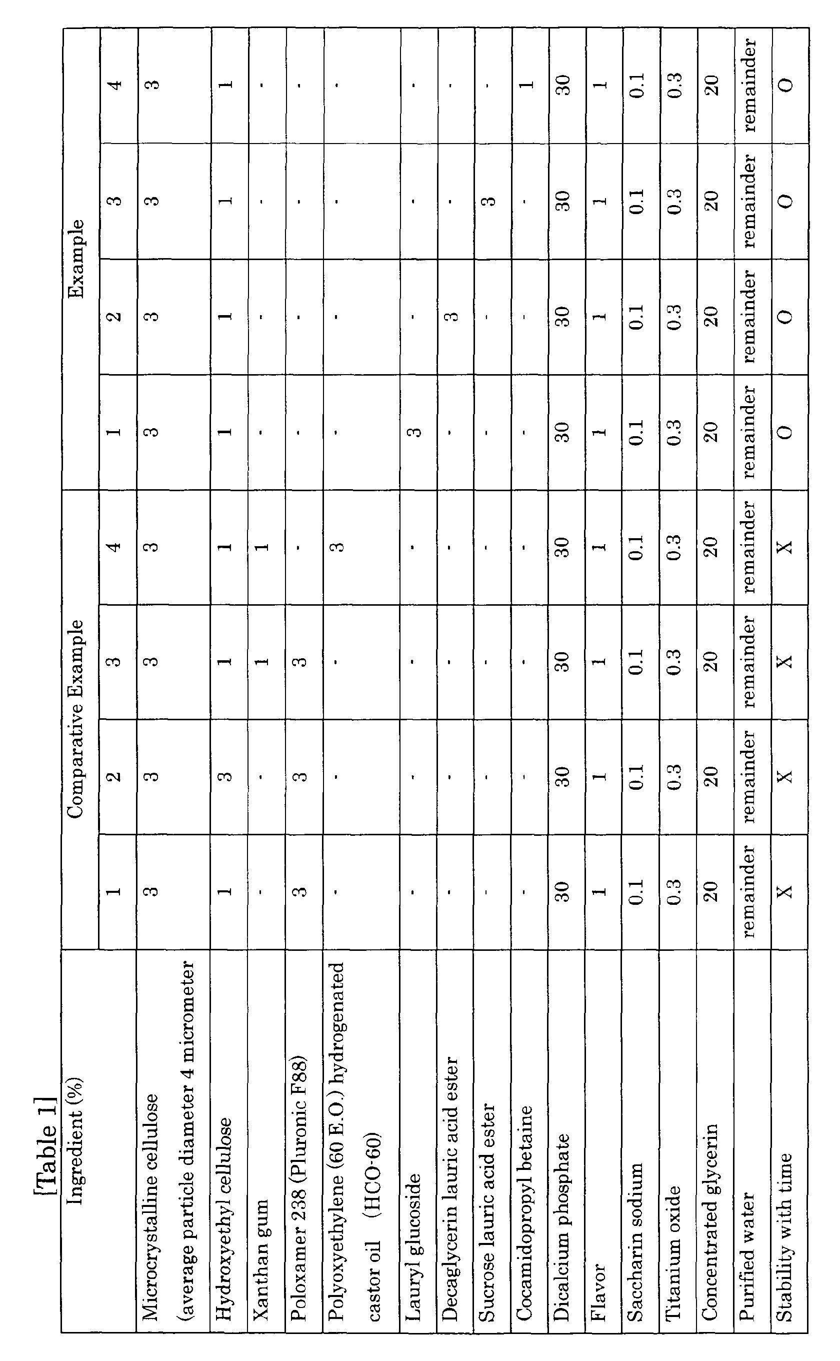

As shown in Table 1, in Comparative Examples 1-3, the oral compositions containing Pluronic F88 as the surface active agent caused solid-liquid separation even when an amount of hydroxyethyl cellulose was increased, or even when xanthan gum as another thickening agent was contained together. In addition, the oral composition caused solid-liquid separation even when HCO-60 was used as the surface active agent.

On the other hand, in Examples 1-4, the oral compositions containing lauryl glucoside, polyglycerin lauric acid ester, sucrose lauric acid ester or cocamidopropyl betaine as the surface active agent, did not cause solid-liquid separation even after one month storage at room temperature, which had excellent stability with time.

### Example 5

An oral composition (toothpaste) of the following formulation was prepared according to the conventional procedures:

| Ingredient Name | Amount (%) |
|---|---|
| Microcrystalline cellulose (average particle diameter 3.7 micrometer) | 3.0 |
| Decyl glucoside | 2.0 |
| Silica | 30.0 |
| Sodium carboxymethyl cellulose | 2.0 |
| Tocopherol acetate | 0.05 |
| Sodium fluoride | 0.2 |
| Flavor | 1.0 |
| Saccharin sodium | 0.1 |
| Titanium oxide | 0.3 |
| Sorbit solution | 30.0 |
| Purified water | remainder |

The oral composition obtained had an excellent shape-holding ability and dispersibility in an oral cavity, did not change a taste of juice after teeth brushing and had excellent stability with time.

### Example 6

An oral composition (toothpaste) of the following formulation was prepared according to the conventional procedures:

| Ingredient Name | Amount (%) |
|---|---|
| Microcrystalline cellulose (average particle diameter 3.7 micrometer) | 3.0 |
| Cocamidopropyl betaine | 0.8 |
| Calcium hydrogenphosphate | 35.0 |
| Cetylpyridinium chloride | 0.1 |
| Hydroxyethyl cellulose | 2.0 |
| Tocopherol acetate | 0.05 |
| Sodium monofluorophosphate | 0.72 |
| Flavor | 1.0 |
| Saccharin sodium | 0.1 |
| Titanium oxide | 0.3 |
| Concentrated glycerin | 15.0 |
| Purified water | remainder |

The oral composition obtained had an excellent shape-holding ability and dispersibility in an oral cavity, did not change a taste of juice after teeth brushing and had excellent stability with time. In addition, the oral composition obtained had an enhanced effect of cetylpyridinium chloride to reside on a tooth surface.

### Example 7

An oral composition (toothpaste) of the following formulation was prepared according to the conventional procedures:

| Ingredient Name | Amount (%) |
|---|---|
| Microcrystalline cellulose (average particle diameter 3.7 micrometer) | 2.0 |
| Sucrose lauric acid ester | 2.0 |
| Calcium pyrophosphate | 35.0 |
| Xanthan gum | 0.5 |
| Sodium monofluorophosphate | 0.72 |
| Flavor | 1.0 |
| Saccharin sodium | 0.1 |
| Titanium oxide | 0.3 |
| Concentrated glycerol | 18.0 |
| Polyethylene glycol | 5.0 |
| Purified water | remainder |

The oral composition obtained had an excellent shape-holding ability and dispersibility in an oral cavity, did not change a taste of juice after teeth brushing and had excellent stability with time.

### Example 8

An oral composition (toothpaste) of the following formulation was prepared according to the conventional procedures:

| Ingredient Name | Amount (%) |
|---|---|
| Microcrystalline cellulose (average particle diameter 3.7 micrometer) | 2.0 |
| Decaglycerin lauric acid ester | 2.0 |
| Calcium carbonate | 25.0 |
| Sodium carboxymethyl cellulose | 1.0 |
| | 1.0 |
| Saccharin sodium | 0.1 |
| Titanium oxide | 0.3 |
| Concentrated glycerin | 10.0 |
| Xylitol | 10.0 |
| Purified water | remainder |

The oral composition obtained had an excellent shape-holding ability and dispersibility in an oral cavity, did not change a taste of juice after teeth brushing and had excellent stability with time.

### Example 9

An oral composition (gel) of the following formulation was prepared according to the conventional procedures:

| Ingredient Name | Amount (%) |
|---|---|
| Microcrystalline cellulose (average particle diameter 3.7 micrometer) | 4.0 |
| Decyl glucoside | 1.0 |
| Sodium fluoride | 0.2 |
| Concentrated glycerin | 40.0 |
| Polyethylene glycol | 5.0 |
| Propylene glycol | 8.0 |
| Flavor | 1.0 |
| Saccharin sodium | 0.1 |
| Disodium hydrogenphophate | 0.12 |
| Sodium dihydrogenphosphate | 0.01 |
| Purified water | remainder |

The oral composition obtained had an excellent shape-holding ability and dispersibility in an oral cavity, did not change a taste of juice after teeth brushing and had excellent stability with time.

### Example 10

An oral composition (gel) of the following formulation was prepared according to the conventional procedures:

| Ingredient Name | Amount (%) |
|---|---|
| Microcrystalline cellulose (average particle diameter 3.7 micrometer) | 5.0 |
| Myristic acid amide propyl betaine | 0.5 |
| Tetraglycerin lauric acid ester | 1.0 |
| Tocopherol acetate | 0.1 |
| Concentrated glycerin | 30.0 |
| Polyethylene glycol | 4.0 |
| 1,3-Butylene glycol | 2.0 |
| Flavor | 1.0 |
| Saccharin sodium | 0.1 |
| Disodium hydrogencitrate | 0.12 |
| Sodium dihydrogencitrate | 0.01 |
| Purified water | remainder |

The oral composition obtained had an excellent shape-holding ability and dispersibility in an oral cavity, did not change a taste of juice after teeth brushing and had excellent stability with time.

### Example 11

An oral composition (toothpaste) of the following formulation was prepared according to the conventional procedures:

| Ingredient Name | Amount (%) |
|---|---|
| Microcrystalline cellulose (average particle diameter 5.8 micrometer) | 0.5 |
| Lauryl glucoside | 2.5 |
| Calcium hydrogenphosphate dihydrate | 40.0 |
| Hydroxyethyl cellulose | 1.0 |
| Flavor | 1.0 |
| Saccharin sodium | 0.2 |
| Sorbitol | 25.0 |
| Purified water | remainder |

The oral composition obtained had an excellent shape-holding ability and dispersibility in an oral cavity, did not change a taste of juice after teeth brushing and had excellent stability with time.

### Example 12

An oral composition (toothpaste) of the following formulation was prepared according to the conventional procedures:

| Ingredient Name | Amount (%) |
|---|---|
| Microcrystalline cellulose (average particle diameter 5.8 micrometer) | 2.0 |
| Decyl glucoside | 1.5 |
| Silicic acid hydrate | 20.0 |
| Carrageenan | 1.0 |
| Flavor | 1.0 |
| Saccharin sodium | 0.1 |
| Sorbitol | 15.0 |
| Concentrated glycerin | 10.0 |
| Purified water | remainder |

The oral composition obtained had an excellent shape-holding ability and dispersibility in an oral cavity, did not change a taste of juice after teeth brushing and had excellent stability with time.

### Example 13

An oral composition (toothpaste) of the following formulation was prepared according to the conventional procedures:

| Ingredient Name | Amount (%) |
|---|---|
| Microcrystalline cellulose (average particle diameter 8.6 micrometer) | 1.0 |
| Cocamidopropyl betaine | 0.8 |
| Silica | 15.0 |
| Aluminum hydroxide | 5.0 |
| Sodium polyacrylate | 0.5 |
| Flavor | 1.0 |
| Saccharin sodium | 0.2 |
| Polyethylene glycol | 5.0 |
| Concentrated glycerin | 10.0 |
| Purified water | remainder |

The oral composition obtained had an excellent shape-holding ability and dispersibility in an oral cavity, did not change a taste of juice after teeth brushing and had excellent stability with time.

### Experiment

### Method of Experiment

### Measurements of an amount of cetylpyridinium chloride residing on hydroxyapatite powder

A 50 mg of hydroxyapatite (DNA Grade Bio-Gel HTP; manufactured by BIO-RAD) was immersed in 2 ml of human saliva, which had been sterilized with ultraviolet rays, at 37 centigrade for 15 hours to allow to form an artificial pellicle on a hydroxyapatite surface. Thereafter, a mixture of hydroxyapatite and human saliva was centrifuged (3000 rpm, 10 min) and a supernatant was discarded. Then, residual hydroxyapatite was immersed at 37 centigrade for 15 minutes in 2 ml of a supernatant of a four times-diluted slurry from each of the oral compositions of Examples 14-18 and Comparative Examples 5-8, in which 0.3 % by weight of cetylpyridinium chloride (CPC) and various amounts and various kinds of thickening agents and surface active agents had been contained. Then, a mixture was centrifuged (3000 rpm, 10 minutes), and a supernatant was discarded. Then, 2 ml of fresh distilled water was added to the residue, the mixture was stirred and centrifuged (3000 rpm, 10 min), and the supernatant was discarded. Again, 2 ml of fresh distilled water was added to the residue, the mixture was stirred and centrifuged (3000 rpm, 10 minutes), and the supernatant was discarded. Next, cetylpyridinium chloride which had adsorbed onto hydroxyapatite was extracted with an extraction solution as described below, and an amount of cetylpyridinium chloride residing on 50 mg of hydroxyapatite was quantitatively measured with high performance liquid chromatography. The results thereof are shown in Table 2.

The extraction solution was prepared by mixing a solution in which 2.88 g of sodium lauryl sulfate had been dissolved per 1 liter of 0.02 M citrate buffer, pH 3 with acetonitrile in a ratio of 1:3.

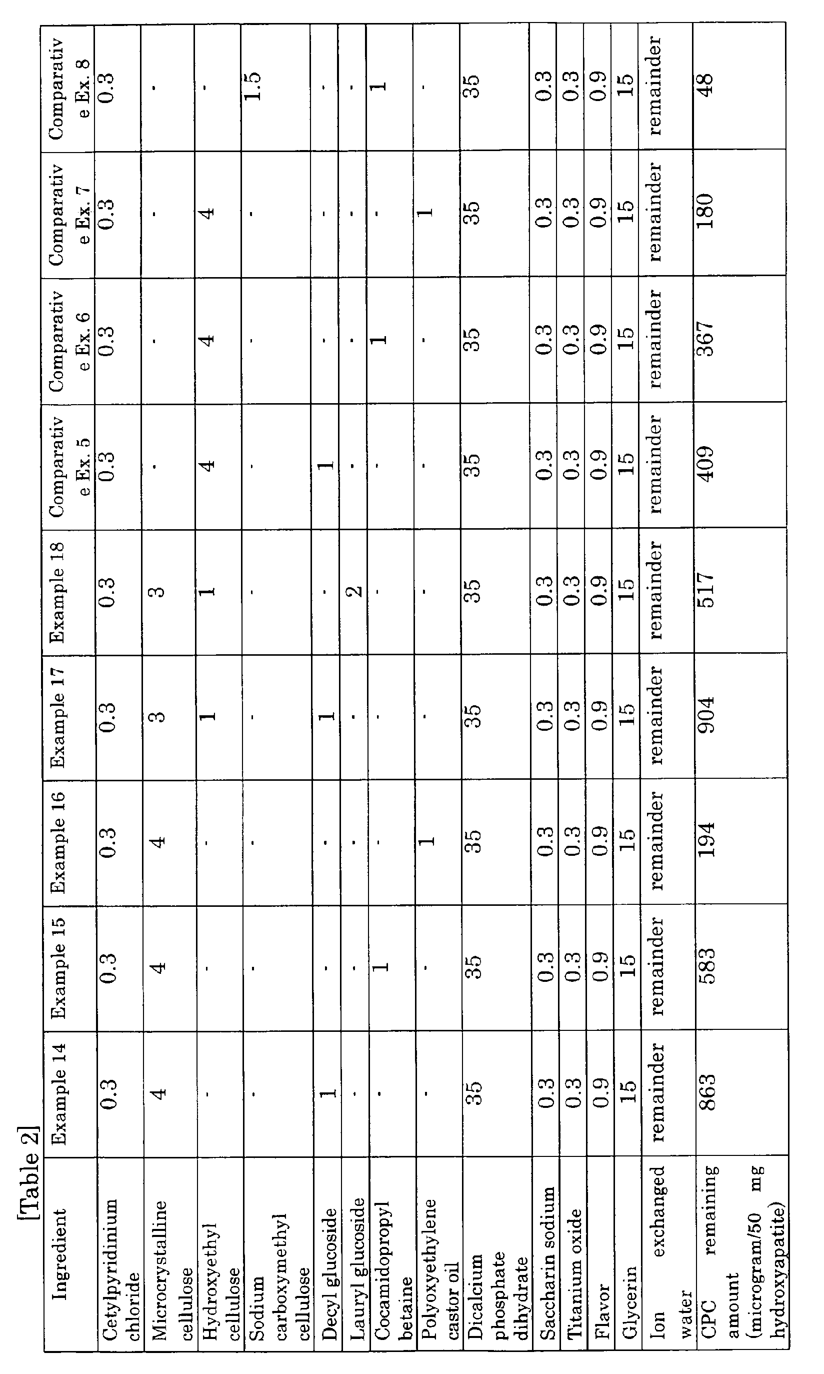

From the results in Table 2, it is found that an amount of cetylpyridinium chloride residing on a tooth surface is significantly increased with the oral compositions of Examples 14-18, in which cetylpyridinium chloride and microcrystalline cellulose have been specifically combined, as compared with those of Comparative Examples 5-8, in which the same amount of cetylpyridinium chloride and other cellulose derivatives have been combined. Moreover, it is also found that alkyl glucoside and betaine are preferable as the surface active agent to be contained in addition to the above ingredients, because they increase an amount of cetylpyridinium chloride residing on a tooth surface, as compared with other surface active agents.

### Example 19

An oral composition (toothpaste) of the following formulation was prepared according to the conventional procedures:

| Ingredient Name | Amount (%) |
|---|---|
| Benzethonium chloride | 0.1 |
| Microcrystalline cellulose (average particle diameter 3.7 micrometer) | 2.0 |
| Triclosan | 0.1 |
| Hydroxypropylmethyl cellulose | 1.0 |
| Lauryl glucoside | 2.0 |
| Calcium carbonate | 40.0 |
| Titanium oxide | 0.2 |
| Saccharin sodium | 0.2 |
| Sorbit solution | 30.0 |
| Flavor | 1.0 |
| Purified water | remainder |

The oral composition obtained could increase an amount of the cationic antimicrobial agent residing on a tooth surface and could effectively prevent an oral cavity disease such as a periodontal disease, dental caries and the like.

### Example 20

An oral composition (toothpaste) of the following formulation was prepared according to the conventional procedures:

| Ingredient Name | Amount (%) |
|---|---|
| Cetylpyridinium chloride | 0.1 |
| Microcrystalline cellulose (average particle diameter 3.7 micrometer) | 3.0 |
| Potassium nitrate | 1.0 |
| Hydroxyethyl cellulose | 2.0 |
| Cocamidopropyl betaine | 1.0 |
| Concentrated glycerin | 10.0 |
| Sorbit solution | 10.0 |
| Titanium oxide | 0.3 |
| Stevioside | 0.2 |
| Sodium benzoate | 0.1 |
| Xylitol | 10.0 |
| Flavor | 0.8 |
| Purified water | remainder |

The oral composition obtained could increase an amount of the cationic antimicrobial agent residing on a tooth surface and could effectively prevent an oral cavity disease such as a periodontal disease, dental caries and the like.

### Example 21

An oral composition (gel) of the following formulation was prepared according to the conventional procedures:

| Ingredient Name | Amount (%) |
|---|---|
| Cetylpyridinium chloride | 0.1 |

| Microcrystalline cellulose | |
|---|---|
| (average particle diameter 3.7 micrometer) | 4.0 |
| Decyl glucoside | 1.0 |
| Concentrated glycerin | 40.0 |
| Polyethylene glycol | 5.0 |
| Propylene glycol | 3.0 |
| Flavor | 1.0 |
| Saccharin sodium | 0.1 |
| Disodium hydrogenphophate | 0.12 |
| Sodium dihydrogenphosphate | 0.01 |
| Purified water | remainder |

The oral composition obtained could increase an amount of the cationic antimicrobial agent residing on a tooth surface and could effectively prevent an oral cavity disease such as a periodontal disease, dental caries and the like.

### Example 22

An oral composition (gel) of the following formulation was prepared according to the conventional procedures:

| Ingredient Name | Amount (%) |
|---|---|
| Chlorhexidine hydrochloride | 0.2 |
| Microcrystalline cellulose (average particle diameter 3.7 micrometer) | 5.0 |
| Myristic acid amide propyl betaine | 0.5 |
| Tetraglycerin lauric acid ester | 1.0 |
| Tocopherol acetate | 0.1 |
| Concentrated glycerin | 30.0 |
| Polyethylene glycol | 4.0 |
| 1,3-Butylene glycol | 2.0 |
| Flavor | 1.0 |
| Saccharin sodium | 0.1 |
| Disodium hydrogencitrate | 0.12 |
| Sodium dihydrogencitrate | 0.01 |
| Purified water | remainder |

The oral composition obtained could increase an amount of the cationic antimicrobial agent residing on a tooth surface and could effectively prevent an oral cavity disease such as a periodontal disease, dental caries and the like.

### Example 23

An oral composition (toothpaste) of the following formulation was prepared according to the conventional procedures:

| Ingredient Name | Amount (%) |
|---|---|
| Benzalkonium chloride | 0.05 |
| Microcrystalline cellulose (average particle diameter 5.8 micrometer) | 0.5 |
| Sucrose myristic acid ester | 4.0 |
| Magnesium carbonate | 5.0 |
| Calcium carbonate | 12.0 |
| Guar gum | 1.0 |
| Flavor | 1.0 |
| Saccharin sodium | 0.2 |
| Concentrated glycerin | 20.0 |
| Purified water | remainder |

The oral composition obtained could increase an amount of the cationic antimicrobial agent residing on a tooth surface and could effectively prevent an oral cavity disease such as a periodontal disease, dental caries and the like.

### Example 24

An oral composition (gel) of the following formulation was prepared according to the conventional procedures:

| Ingredient Name | Amount (%) |
|---|---|
| Chlorhexidine gluconate | 0.2 |
| Microcrystalline cellulose (average particle diameter 8.6 micrometer) | 5.0 |
| Myristyl glucoside | 4.0 |
| Hydroxypropylmethyl cellulose | 1.0 |
| Flavor | 0.5 |
| Saccharin sodium | 0.2 |
| Concentrated glycerin | 20.0 |
| Propylene glycol | 3.0 |
| Purified water | remainder |

The oral composition obtained could increase an amount of the cationic antimicrobial agent residing on a tooth surface and could effectively prevent an oral cavity disease such as a periodontal disease, dental caries and the like.

According to the first aspect of the present invention, an oral composition can be provided, which has an excellent shape-holding ability and dispersibility in an oral cavity, does not change a taste of juice after teeth brushing, and particularly, excellent stability with time, or which has an enhanced ability of a cationic antimicrobial agent to reside on a tooth surface.

Moreover, according to the second aspect of the present invention, an oral composition can be provided, which can significantly increase an amount of a cationic antimicrobial agent residing on a tooth surface and effectively prevent an oral cavity disease such as a periodontal disease, dental caries and the like.

## Claims

1. An oral composition comprising microcrystalline cellulose, and one or more surface active agents selected from the group consisting of alkyl glucoside, polyglycerin fatty acid ester, sucrose fatty acid ester and betaine.

2. The oral composition of according to claim 1, wherein the microcrystalline cellulose is contained at 0.2-10 % by weight.

3. The oral composition according to claim 1 or 2, wherein the surface active agent is alkyl glucoside.

4. The oral composition according to claim 3, wherein an alkyl chain of the alkyl glucoside is C8-C16 in length.

5. The oral composition according to claim 1 or 2, wherein the surface active agent is polyglycerin fatty acid ester or sucrose fatty acid ester.

6. The oral composition according to claim 5, wherein an alkyl chain of a fatty acid portion of the polyglycerin fatty acid ester or the sucrose fatty acid ester is C8-C16 in length.

7. The oral composition according to claim 1 or 2, wherein the surface active agent is betaine.

8. The oral composition according to claim 7, wherein the betaine is fatty acid amide propyl betaine.

9. The oral composition according to claim 8, wherein an alkyl chain of a fatty acid portion of the fatty acid amide propyl betaine is C8-C16 in length.

10. The oral composition according to any one of claims 1-9, further comprising a cationic antimicrobial agent.

11. An oral composition comprising a cationic antimicrobial agent and microcrystalline cellulose.

12. The oral composition according to claim 11, wherein the cationic antimicrobial agent is a quaternary ammonium salt.

13. The oral composition according to claim 11, wherein the cationic antimicrobial agent is a biguanide antimicrobial agent.

14. The oral composition according to claim 11, wherein the cationic antimicrobial agent is one or more selected from the group consisting of cetylpyridinium chloride, benzalkonium chloride, benzethonium chloride, chlorhexidine hydrochloride and chlorhexidine gluconate.

15. The oral composition according to any one of claims 11-14, wherein the cationic antimicrobial agent is contained at 0.001-10 % by weight.

16. The oral composition according to any one of claims 11-15, wherein the microcrystalline cellulose is contained at 0.2-10 % by weight.

17. The oral composition according to any one of claims 11-16, further comprising one or more surface active agents selected from nonionic and amphoteric surface active agents.

18. The oral composition according to claim 17, wherein the surface active agent is alkyl glucoside having an alkyl chain of C8-C16 in length.

19. The oral composition according to claim 17, wherein the surface active agent is fatty acid amide propyl betaine having an alkyl chain of a fatty acid portion of C8-C16 in length.
